# EUROPEAN PATENT APPLICATION

(11) **EP 3 725 247 A1**
(43) Date of publication of application: **21.10.2020**
(21) Application number: 18888832.5
(22) Date of filing: 13.12.2018
(51) Int. Cl.: A61B 17/70

(54) **BACKBONE FIXING SYSTEM**

(30) Priority: 15.12.2017 JP 2017240192
(71) Applicant: MIZUHO Corporation, Tokyo 113-0033 (JP)
(72) Inventor: EBARA Sohei, Kanagawa 251-0047 (JP)
(74) Representative: SSM Sandmair
(86) International application number: PCT/JP2018/045888
(87) International publication number: WO 2019/117251

(57) **Abstract**

[Task]

Provided is the spinal fixation system that facilitates surgical operations and improves reliability in stable fixation to a spine.

[Means to solve the task]

A spinal fusion system 1A includes a connector 6A (6A') detachably connected to a rod 3, and a hook member 7A (7A) connected to the connector 6A (6A') and engaged with a vertebral arch. Thereby, in particular, reliability in stable fixation to the spine can be improved.

## Description

### Technical field

The present invention relates to, for example, a spinal fusion system for correcting and fixing spinal deformity.

### Background Art

In its normal condition, a spine is generally straight when viewed from the back, cervical vertebrae and lumbar vertebrae curve forward when viewed from the side, and thoracic vertebrae and sacral vertebrae curve backward. Accordingly, the spine shows an approximately S-shaped appearance.

Spinal deformity includes diseases such as scoliosis or kyphosis. Scoliosis is the disease in which a spine is curved laterally and twisted. Kyphosis is the disease in which the angle of thoracic kyphosis becomes extremely large, or lumbar lordosis is lost so as to be deformed toward kyphosis.

In the treatment of such types of spinal deformities, spinal deformity correction and fusion surgeries are generally conducted. Spinal deformity correction and fusion surgeries are operations for first correcting deformed spines and returning them to a normal state or a state closer thereto and then fixing the deformed spines. Spinal deformity correction and fusion surgeries involve a posterior correction and fusion surgery or an anterior correction and fusion surgery. In particular, the posterior correction and fusion surgery is conducted as follows. A patient is positioned on an operation table in a prone position. Then, an operative wound or a percutaneous operative wound using minimally invasive techniques is placed in the exact middle of the patient's back, and the posterior elements of the spine are unfolded. Subsequently, a later-explained spinal deformity correction and fusion system is installed to the spine so as to three-dimensionally correct the spinal deformity. The spine is fixed in that condition.

Generally, the spinal deformity correction and fusion system employs: a plurality of screws that are to be screwed into a vertebral body through the pedicle of each vertebra of a spine; a plurality of hook members that are to be hooked on the pedicle or transverse process, etc. of each vertebra; a pair of rods that are connected to the top-opened groove of each screw and each hook member and extend along the axis of the spine or arranged with spaces in a crosswise direction; and a coupler that bridges each rod in order to increase rigidity in twisting directions with respect to the pair of rods (see Patent Document 1). Further, a set screw is used for connecting the rod to each screw and hook member. When connecting the rod to the top-opened groove of the screw and the hook member, the rod is first engaged within the top-opened groove of the screw and the hook member, and then the set screw is screwed into female threads provided on the inner wall surface of the top-opened groove. By pressing the rod to the bottom surface of the top-opened groove, the rod is connected to the screw and the hook member.

### Prior Art

### Patent Literature

Patent Literature 1: Japanese Patent No. 4907352

### Summary of Invention

### Problems to be Solved by Invention

However, in the conventional spinal deformity correction and fusion system, the coupler is provided so as to bridge each rod, so that rigidity in twisted directions relative to the pair of rods is secured. In this regard, there is some concern that each rod may be loosened in a sliding direction relative to each screw and each hook member, or each screw may be loosened in a drawn direction relative to the vertebral body of each screw. Accordingly, in the conventional spinal deformity correction and fusion system, reliability in stable correction and fusion (fixation force) to the spine has been lacking. Further, in the conventional system, operative procedures for three-dimensionally correcting spinal deformity including twisting becomes extremely difficult and complicated, and as a result, an operative duration extends while the burden on patients becomes large.

The present invention has been made in view of the above, and its object is to provide a spinal fusion system that facilitates operative processes and improves reliability in stable fixation to a spine.

### Means for Solving Problems

### (Aspects of Invention)

The aspects of the invention described below exemplify the configuration of the present invention, and are described separately by items in order to facilitate the understanding of various configurations of the present invention. Each item does not limit the technical scope of the present invention, and, while taking into consideration the best mode for carrying out the invention, partial replacement and deletion of some of the components of each configuration, or further addition of other configurations should be included in the technical scope of the present invention.
(1) It is a spinal fusion system including: each supporting element that supports each vertebra constituting a spine; and a rod that extends along an axial direction of the spine and is connectable to the supporting element, the spinal fusion system further including: a connector that is detachably connectable to the rod; and a hook member that is connectable to the connector and engageable with a vertebral arch (corresponding to the invention recited in claim 1).
   In the spinal fusion system recited in the above (1), since the system is provided with the hook member connected to the rod via the connector and engaged with a vertebral arch, fixation force to a spine can be strengthened by the spinal fusion system. Note that the hook member may be engaged with, for example, a lamina.
   More specifically, in general spinal deformity correction and fusion systems, by using a set screw, a rod is pressed against the top-opened groove of, for example, a screw or a hook member, which is a supporting element. Through this, the rod is connected to the screw or the hook member so as to correct and fix spinal deformity. On the other hand, in the spinal fusion system according to the above (1), since the system is provided with the hook member connected to the rod via the connector and engaged with the vertebral arch, when an external force is applied to the rod, it is possible that the external force can be dispersed to the vertebral arch via the connector and the hook member. As a result, the loosening of the rod in the sliding direction with respect to the screw and the hook member can be suppressed. Moreover, the loosening of the screw in the pull-out direction with respect to the vertebral body can be suppressed.
(2) In the spinal fusion system of (1), it is characterized in that the connector has an arm portion that extends in a direction approximately orthogonal to an axial direction of the rod, and the hook member is detachably installed at an optional position along an axial direction of the arm portion (corresponding to the invention recited in claim 2).
   In the spinal fusion system of (2), during an operation, the hook member can be easily engaged with the vertebral arch according to a distance between the rod and the vertebral arch in a crosswise direction. Moreover, the hook member can be installed to the arm portion of the connector. Consequently, surgical procedures using the spinal fusion system is facilitated.
(3) In the spinal fusion system of (2), the hook member includes an arm receiving portion that has a groove portion in which to receive the arm portion of the connector, and a hook portion that is connectable to the arm receiving portion and is engageable with the vertebral arch, wherein the arm receiving portion is configured to be integrally connectable with the hook portion at an optional position in such a manner that a direction where the groove portion extends is allowed to be optionally oriented (corresponding to the invention of claim 3).
   In the spinal fusion system of (3), the arm receiving portion of the hook member is connected to the hook portion in such a manner that the direction where the groove portion extends can be optionally oriented. Accordingly, the arm portion of the connector can be easily engaged within the arm receiving portion of the hook member during operation. Surgical procedures using the spinal fusion system can be thus facilitated.
(4) In the spinal fusion system of (2) or (3), it is characterized in that the connector is provided with a rod receiving portion that is connectable to the arm portion and has a groove portion in which to receive the rod, and the rod receiving portion is configured to be integrally connectable with the arm portion at an optional position in such a manner that a direction where the groove portion extends is allowed to be optionally oriented (corresponding to the invention of claim 4).
   In the spinal fusion system of (4), the rod receiving portion of the connector is connected to the arm portion in such a manner that the direction where the groove portion extends can be oriented in arbitrarily directions. Accordingly, the rod can be easily engaged within the rod receiving portion of the connector during operation. Surgical procedures using the spinal fusion system can be thus facilitated.
(5) In the spinal fusion system of (1), it is characterized in that the connector has an arm portion that extends in a direction approximately orthogonal to an axial direction of the rod, and the hook member is slidably connectable along an axial direction of the arm portion (corresponding to the invention of claim 5)
   In the spinal fusion system of (5), the hook member is slidably connected along the axial direction of the arm portion of the connector. Accordingly, the hook member can be easily engaged with the vertebral arch according to a distance between the rod and the vertebral arch in a crosswise direction. Surgical procedures using the spinal fusion system can be thus facilitated.
(6) In the spinal fusion of any one of (2) to (5), it is characterized in that the pair of rods are arranged on both left and right sides of a spinous process of each vertebral arch, and both longitudinal ends of the arm portion are each detachably connectable to the pair of rods (corresponding to the invention of claim 6).
   In the spinal fusion system of (6), the rigidity of the pair of rods in twisting directions can be enhanced with the connector.
(7) It is a spinal fusion system including: a rod that extends along an axial direction of a spine; and a first hook member that is detachably installed to the rod and is engageable with a vertebral arch, wherein the first hook member includes a rod receiving portion that has a groove portion in which to receive the rod, and a hook portion that is connectable to the rod receiving portion and is engageable with the vertebral arch, and the rod receiving portion is configured to be integrally connectable to the hook portion at an optional position in such a manner that a direction where the groove portion extends is allowed to be optionally oriented (corresponding to the invention of claim 7).
   In the spinal fusion system of (7), the rod receiving portion of the first hook member is connected to the hook portion in such a manner that the direction where the groove portion extends can be oriented in arbitrarily directions. Accordingly, the rod can be easily engaged within the rod receiving portion of the first hook member during operation. Surgical procedures using the spinal fusion system can be thus facilitated.
(8) In the spinal fusion system of (7), it is characterized in that the system includes: a connector that is detachably connectable to the rod, and a second hook member that is connectable to the connector and engageable with a vertebral arch (corresponding to the invention of claim 8).
   In the spinal fusion system of (8), since the system is provided with the second hook member connected to the rod via the connector and engaged with a vertebral arch, it is possible to strengthen a stable fixing force to a spine.
(9) In the spinal fusion system of (8), the second hook member includes an arm receiving portion having a groove in which to receive an arm portion constituting the connector, and a hook portion that is engageable with the vertebral arch, wherein the arm receiving portion is configured to be integrally connectable with the hook portion at an optional position in such a manner that a direction where the groove portion extends is allowed to be optionally oriented.
   In the spinal fusion system of (9), the arm portion of the connector can be easily engaged with the arm receiving portion of the second hook member during operation. A surgical procedure using the spinal fixation system can be thus facilitated.
(10) In the spinal fusion system of (9), it is characterized in that the first hook member and the second hook member are commonly configured.
   In the spinal fusion system of (10), since the first hook member and the second hook member can be made as common parts, an operator does not need to select the hook member during operation. The operation time can be thus reduced.

### EFFECT OF THE INVENTION

In the spinal fusion system according to the present invention, operative procedures can be facilitated, and reliability in stable fixation to a spine can be improved.

### BRIEF DESCRIPTION OF DRAWINGS

FIG. 1 is the rear view of a spinal fusion system according to the first embodiment.
FIG. 2 is the perspective view of a screw adopted in the spinal fusion system according to the first embodiment.
FIG. 3 is the sectional view of a rod receiving portion of the screw of FIG. 2;
FIG. 4 is a view showing a state where the screw of FIG. 2 is screwed into a vertebra.
FIG. 5 is the perspective view of a first hook member adopted in the spinal fusion system according to the first embodiment.
FIG. 6 is a perspective view showing a state where the first hook member of FIG. 5 is engaged with the pedicle of a vertebral arch;
FIG. 7 is the perspective view of a second hook member adopted in the spinal fusion system according to the first embodiment.
FIG. 8 is a perspective view showing a state where the second hook member of FIG. 7 is engaged with the lamina of the vertebral arch;
FIG. 9 is the perspective view of a second hook member according to another embodiment.
FIG. 10 is the sectional view of the second hook member of FIG. 9;
FIG. 11 is the perspective view of a first hook member according to another embodiment.
FIG. 12 is the perspective view of a connector adopted in the spinal fusion system according to the first embodiment.
FIG. 13 is the perspective view of a connector according to another embodiment.
FIG. 14 is the sectional view of the connector of FIG. 13.
FIG. 15 is the rear view of a spinal fusion system according to the second embodiment.
FIG. 16 is the perspective view of a connector and a second hook member adopted in the spinal fusion system according to the second embodiment.
FIG. 17 is a sectional view showing a state where a rod is integrally connected to a rod receiving portion of the connector of FIG. 16.

### EMBODIMENTS FOR CARRYING OUT THE INVENTION

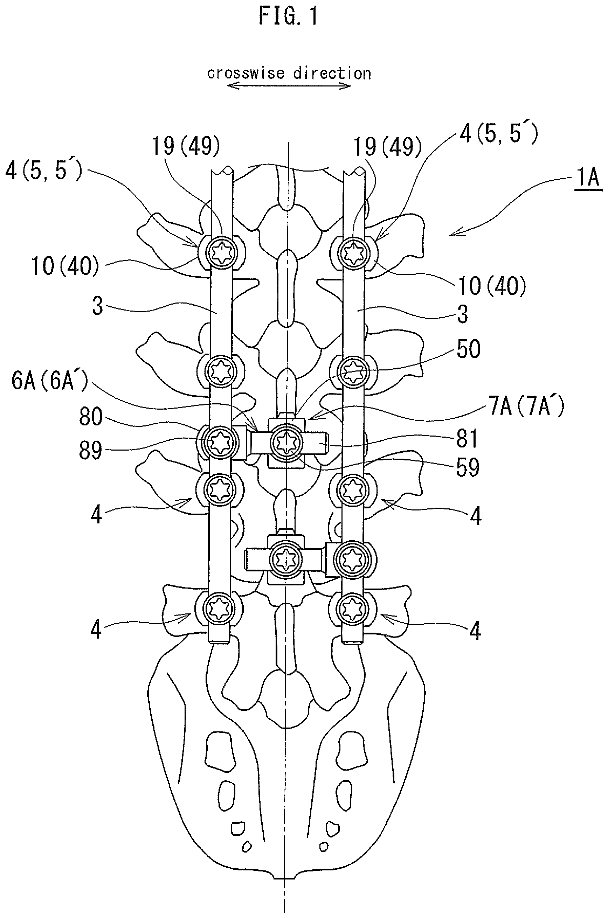

Hereinafter, embodiments for carrying out the present invention will be described in detail with reference to FIG. 1 to FIG. 17.

Spinal fusion systems 1A, 1B according to the first and second embodiments of the present invention adopt a spinal deformity correction and fusion system for correcting and fixing spinal deformity such as scoliosis and kyphosis.

First, the spinal fusion system 1A according to the first embodiment will be described in detail with reference to FIG. 1 to FIG. 14. As shown in FIG. 1, the spinal fusion system 1A according to the first embodiment includes: a pair of rods 3, 3 extending along the axial direction of a spine; a screw 4 (supporting element) detachably attached to the rod 3 and screwed into a vertebral body via the pedicle of each vertebra; a first hook member 5 (supporting element) detachably attached to the rod 3 and engaged with the pedicle or transverse process of each vertebra; a connector 6A detachably attached to the rod 3, and a second hook member 7A connected to the connector 6A and engaged with the lamina of a vertebral arch.

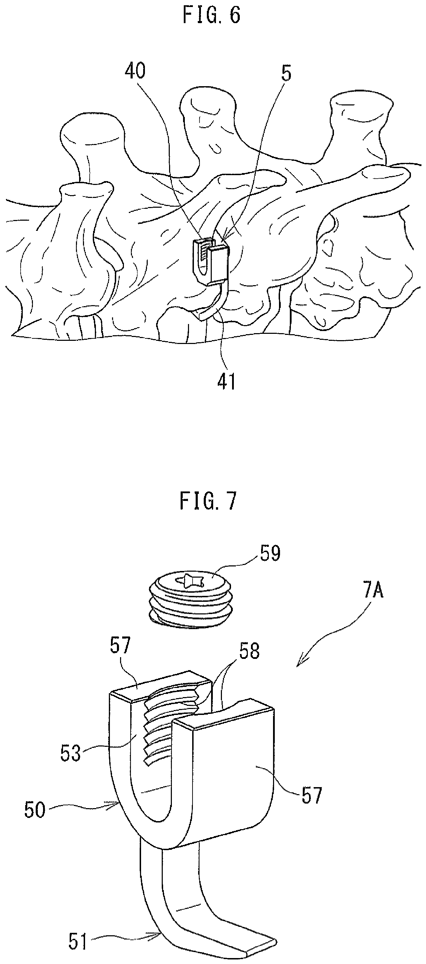

Note that the first hook member 5 will be adopted as needed. That is, when dealing with cases such as scoliosis, the screw 4 is normally adopted. However, for example, when the outer diameter of pedicle is small and the screw 4 cannot be screwed into the pedicle, the first hook member 5 may be adopted, the first hook member 5 being hookedly engaged with the pedicle or transverse process (pedicle in FIG. 6). The rod 3, the screw 4, the first hook member 5, the connector 6A and the second hook member 7A are formed of a highly biocompatible material such as titanium alloy.

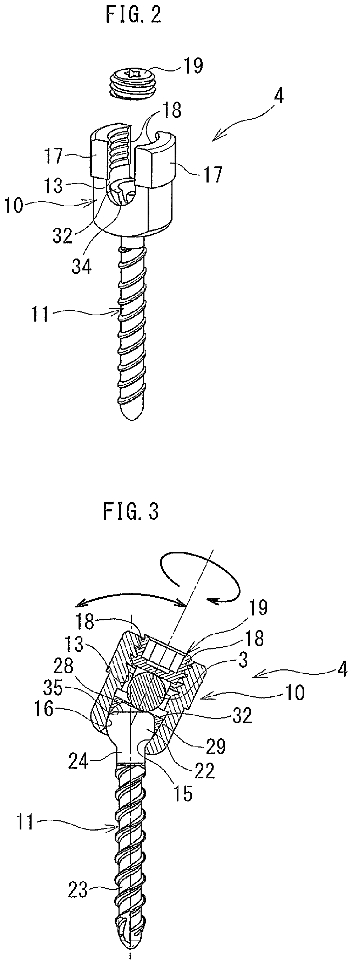

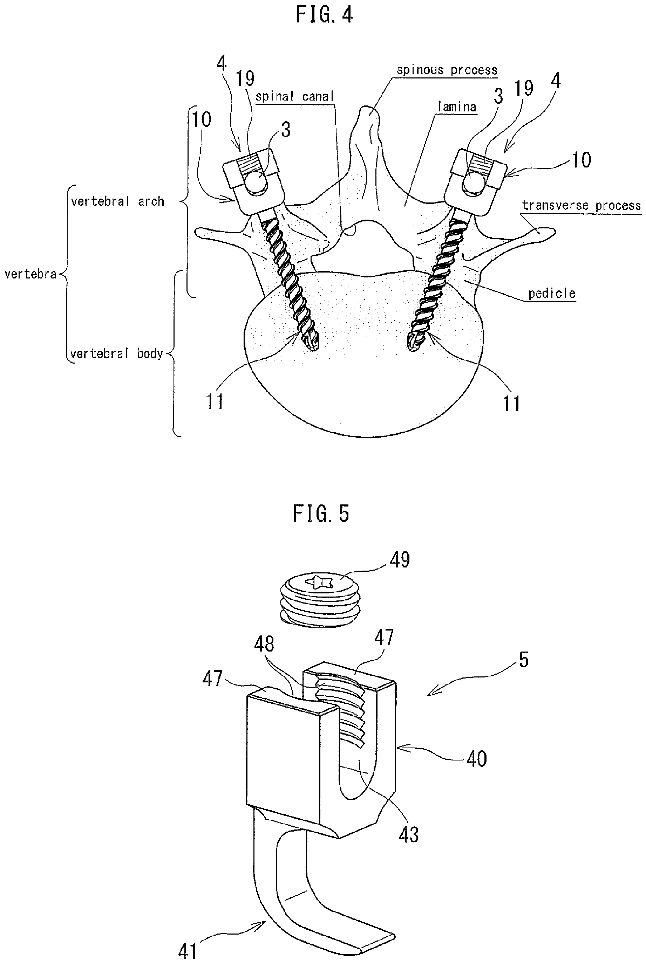

As shown in FIG. 1 and FIG. 4, each rod 3 extends along the axial direction of the spine, the rod being arranged on both left and right sides of the spine, by defining the spinous process of each vertebral arch as a boundary. The rod 3 has a substantially circular cross section. The outer diameter and the length of the rod 3 are appropriately selected and determined by an operator, based on a corrective range for spinal deformity and the like. As can be seen from FIG. 4, the screw 4 is screwed into the vertebral body, from the posterior of the spine through the pedicle of each vertebra. The screw 4 is generally called as a pedicle screw. As shown in FIG. 2 and FIG. 3, the screw 4 includes a rod receiving portion 10 having a groove portion 13 for receiving the rod 3, and a screw portion 11 coupled to the rod receiving portion 10 and screwed into the vertebral body through the pedicle of the vertebra.

The rod receiving portion 10 is formed in a block shape. In the rod receiving portion 10, the U-shaped groove portion 13, whose one end surface opposite to the screw portion 11 side is opened, is formed along the axial direction of the rod 3. The rod 3 is then received in the groove portion 13. An insertion hole 15 is formed in such a manner as to pass through the bottom portion of the groove portion 13. An engagement head portion 22 and a pressing member 32 of the screw portion 11, which will be described later, are engaged in the insertion hole 15. A locking spherical surface 16 is formed on the inner wall surface of the insertion hole 15, the locking spherical surface locking a spherical surface 29 provided on the engagement head portion 22 of the screw portion 11. Further, in the rod receiving portion 10, female threads 18 are formed on each inner wall surface of walls 17, 17 facing each other, by defining the groove portion 13 as a boundary. A set screw 19 is screwed into the female threads 18. Then, the set screw 19 moves the rod 3, which is located in the rod receiving portion 10, toward the bottom of the groove portion 13 so as to push down a later-explained pressing member 32 arranged in the rod receiving portion 10. With this, the rod can be integrally connected to the screw 4.

The screw portion 11 extends from the other end surface of the rod receiving portion 10. The screw portion 11 includes the engagement head portion 22 engaged in the insertion hole 15 of the rod receiving portion 10, and male threads 23 integrally connected to the engagement head portion 22 via a connection portion 24. The engagement head portion 22 of the screw portion 11 has a plane portion 28 formed on the top thereof and the spherical surface 29 formed continuously from the plane portion 28. In the rod receiving portion 10, relative to the screw portion 11, the direction where the U-shaped groove portion 13 extends can be optionally oriented. Further, the rod receiving portion 10 is configured to be integrally connectable with the screw portion 11 at optional positions. This connective structure will be described in detail. In the insertion hole 15 of the rod receiving portion 10, the pressing member 32 is slidably engaged in its axial direction. The pressing member 32 has a substantially cylindrical shape and has a cutout portion 34, which is partially positioned in the circumferential direction of the pressing member 32, the cutout portion 34 extending in the axial direction of the pressing member 32 and connecting the inside with the outside of the pressing member 32. The inner wall surface of the pressing member 32 is formed with a pressing spherical surface 35. The pressing spherical surface 35 of the pressing member 32 is adapted to abut to the spherical surface 29 of the engagement head portion 22 of the screw portion 11.

Thus, the rod receiving portion 10 is connected to the screw portion 11, the rod receiving portion 10 being rotatable around the shaft direction of the female threads 18. Further, the rod receiving portion 10 is connected to the screw portion 11 so as to be swingable in all directions within a predetermined angle. Then, the set screw 19 is screwed into the female threads 18 of the rod receiving portion 10, and the rod 3 is moved toward the side of the screw portion 11 together with the pressing member 32. Accordingly, the pressing spherical surface 35 of the pressing member 32 presses down the spherical surface 29 of the engagement head portion 22 of the screw portion 11 while the spherical surface 29 of the engagement head portion 22 presses down the locking spherical surface 16 of the insertion hole 15. By this, in the rod receiving portion 10, the direction where the groove portion 13 extends is optionally positioned relative to the screw portion 11. The rod receiving portion 10 is thus integrally connected to the screw portion 11. Simultaneously with the above, the rod 3 is integrally connected to the rod receiving portion 10 of the screw 4. Considering the screw 4 adopted in the present embodiments, the rod receiving portion 10 is connected to the screw portion 11 in such a manner that the direction where the U-shaped groove portion 13 extends can be optionally oriented. In addition to the above, the following screw may be adopted. That is, the rod receiving portion 10 is connected to the screw portion 11 in such a manner that the rod receiving portion 10 is swingable within a certain angle range along the direction in which the U-shaped groove portion 13 extends.

As shown in FIG. 5 and FIG. 6, and also referring to FIG. 1, the first hook member 5 is hooked to and engaged with pedicle or transverse process (pedicle in FIG. 6) of a vertebral arch. The first hook member 5 includes a rod receiving portion 40 having a groove portion 43 for receiving the rod 3, and a first hook portion 41 integrally connected to the rod receiving portion 40. The rod receiving portion 40 is formed in a block shape. In the rod receiving portion 40, the U-shaped groove portion 43, in which one end surface thereof opposite to the first hook portion 41 side is opened, is formed along the axial direction of the rod 3. The rod 3 is received in the U-shaped groove 43. In the rod receiving portion 40, each inner wall surface of wall portions 47, 47 facing each other in the groove portion 43 has female threads 48. A set screw 49 is screwed into the female threads 48.

The set screw 49 presses the rod 3 located in the rod receiving portion 40 against the bottom surface of the groove portion 43 of the rod receiving portion 40 so as to integrally connect the rod 3 to the first hook member 5. The first hook portion 41 extends in substantially an L shape from the other end surface of the rod receiving portion 40. The tip of the first hook portion 41 extends in the same direction where the groove portion 43 provided in the rod receiving portion 40 extends. With this first hook portion 41, it is possible to engage the pedicle or the transverse process of a vertebral arch. On the inner surface of the first hook portion 41, in order to increase frictional force against the pedicle or the transverse projection of the vertebral arch, at least one sharp-pointed convex portion or uneven portion, etc. may be formed in a longitudinal or lateral direction.

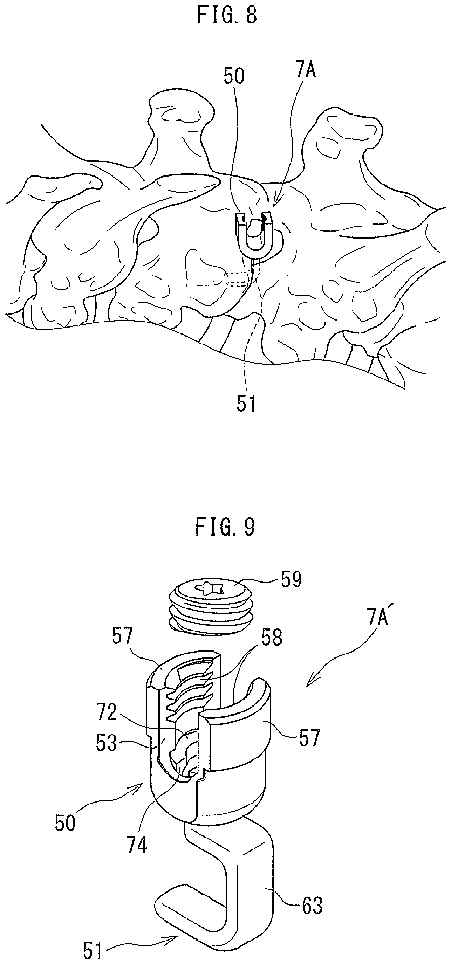

As shown in FIG. 7 and FIG. 8, and also referring to FIG. 1, the second hook member 7A conducts engagement in such a manner as to hook the lamina of a vertebral arch from the inside of a spinal canal. The second hook member 7A includes an arm receiving portion 50 having a groove portion 53 for receiving an arm portion 81 of the connector 6A described later, and a second hook portion 51 integrally connected to the arm receiving portion 50. The arm receiving portion 50 is formed in a block shape. In the arm receiving portion 50, the U-shaped groove portion 53, in which one end surface thereof opposite to the second hook portion 51 side is opened, is formed. The groove portion 53 is formed along the axial direction of the arm portion 81 of the connector 6A, that is, along the direction substantially orthogonal to the axial direction of the rod 3. The arm portion 81 of the connector 6Ais received in the U-shaped groove portion 53.

In the arm receiving portion 50, each inner wall surface of wall portions 57, 57 facing each other in the groove portion 53 has female threads 58. A set screw 59 is screwed into the female threads 58. The set screw 59 presses the arm portion 81 of the connector 6A located in the arm receiving portion 50 against the bottom surface of the groove portion 53 of the arm receiving portion 50. Thus, not only the arm portion 81 but also the connector 6Ais integrally connected to the second hook member 7A. The second hook portion 51 extends in a substantially L shape, from the other end surface of the arm receiving portion 50. The tip of the second hook portion 51 extends in the direction substantially orthogonal to the direction in which the groove portion 53 provided in the arm receiving portion 50 extends. In other words, the tip of the second hook portion 51 extends toward one of the wall portions 57 of the arm receiving portion 50. The second hook portion 51 conducts engagement in such a manner as to hook the lamina of a vertebral arch from the inside of a spinal canal. In order to increase frictional force against the lamina of the vertebral arch on the inner surface of the second hook portion 51, at least one sharp-pointed convex portion or uneven portion, etc. may be formed in a longitudinal or lateral direction.

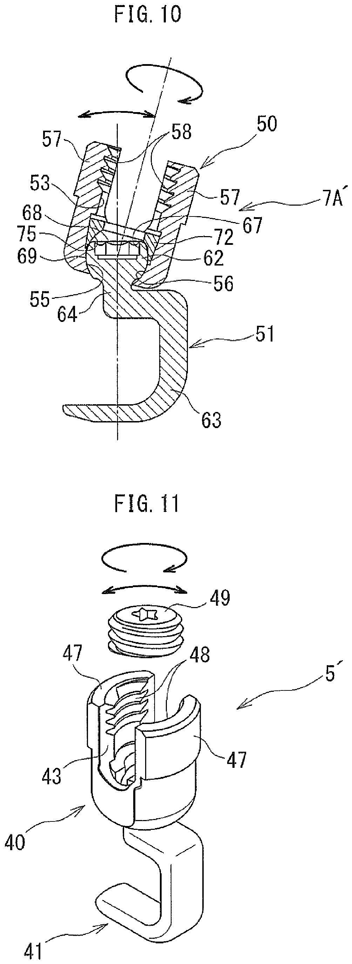

Next, a second hook member 7A' according to another embodiment will be described with reference to FIG. 9 and FIG. 10. Considering the second hook member 7A' according to another embodiment, in the arm receiving portion 50, relative to the second hook portion 51, the direction where the U-shaped groove portion 53 extends can be optionally oriented. Further, the arm receiving portion 50 is configured to be integrally connectable with the second hook portion 51 at optional positions. This connection structure will be described in detail. The second hook portion 51 includes a U-shaped hook portion 63 to be hooked to and engaged with lamina, and an engagement head portion 62 integrally connected to the end portion of the arm receiving portion 50 side of the U-shaped hook portion 63 through a connecting portion 64 and engaging with the arm receiving portion 50. The engagement head portion 62 is formed in a spherical shape, and a plane portion 68 is formed on the top thereof. Note that the plane portion 68 has a polygonal concaved portion 67 having a polygonal shape in a plan view.

On the other hand, an insertion hole 55 is formed through the bottom of the groove portion 53 of the arm receiving portion 50. The engagement head portion 62 of the second hook portion 51 is engaged in the insertion hole 55. The inner wall surface of the insertion hole 55 has a locking spherical surface 56 for locking a spherical surface 69 provided on the engagement head portion 62 of the second hook portion 51. Within the insertion hole 55, a pressing member 72 is engaged slidably in the axial direction. The pressing member 72 has a substantially cylindrical shape and has a cutout portion 74, which partially positioned in the circumferential direction, extends in the axial direction and connects the inside with the outside of the pressing member 72. A pressing spherical surface 75 is formed on the inner wall surface of the pressing member 72. The pressing spherical surface 75 of the pressing member 72 comes into contact with the spherical surface 69 of the engagement head portion 62 of the second hook portion 51. The arm receiving portion 50 and the second hook portion 51 are fitted and assembled in the insertion hole 55 of the arm receiving portion 50 from the engagement head portion 62 side of the second hook portion 51.

Thereby, the arm receiving portion 50 is connected to the second hook portion 51, the arm receiving portion 50 being rotatable around the axial direction of the female threads 58 of the arm receiving portion 50. Further, the arm receiving portion 50 is connected to the second hook portion 51 so as to be swingable in all directions within a predetermined angle range. Then, the set screw 59 is screwed into the female threads 58 of the arm receiving portion 50, and the arm portion 81 of the connector 6Ais moved toward the second hook portion 51 side together with the pressing member 72. With this, the pressing spherical surface 75 of the pressing member 72 presses the spherical surface 69 of the engagement head portion 62 of the second hook portion 51 while the spherical surface 69 of the engagement head portion 62 presses the locking spherical surface 56 of the insertion hole 55. Accordingly, the arm receiving portion 50 is integrally connected to the second hook portion 51 in such a manner that the direction where the groove portion 53 extends is arbitrarily positioned relative to the second hook portion 51. Then, at the same time, the arm portion 81 of the connector 6A described later is integrally connected to the arm receiving portion 50 of the second hook member 7A'.

In the first hook member 5, it is possible to adopt the same embodiment as the second hook member 7A' according to another embodiment. In short, as shown in FIG. 11, in the first hook member 5' according to another embodiment, in the rod receiving portion 40, relative to the first hook portion 41, the direction where the U-shaped groove portion 43 extends can be optionally oriented. Further, the rod receiving portion 40 is configured to be integrally connectable with the first hook portion 41 at optional positions. Since the connection structure is the same as that of the second hook member 7A' according to another embodiment, the description is omitted here.

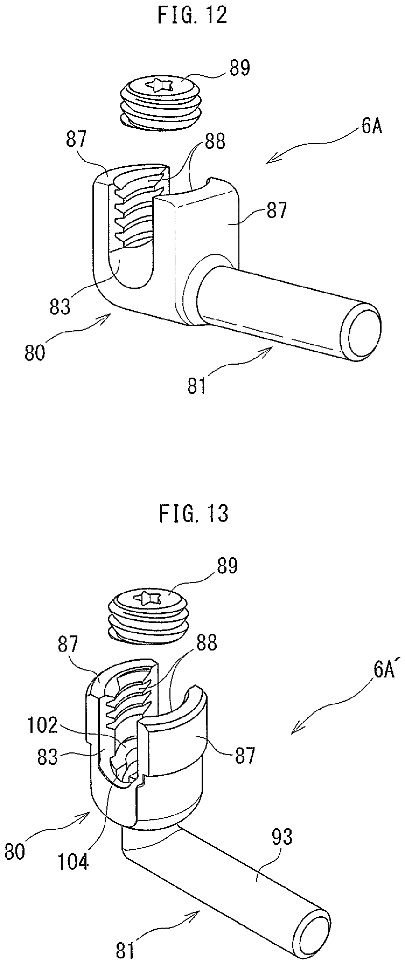

As shown in FIG. 12, also referring to FIG. 1, the connector 6A includes a rod receiving portion 80 having a groove portion 83 for receiving the rod 3 and an arm portion 81 integrally connected to the rod receiving portion 80. The rod receiving portion 80 of the connector 6A is formed substantially in the same manner as the rod receiving portion 40 of the first hook member 5. The rod receiving portion 80 of the connector 6A is formed in a block shape. In the rod receiving portion 80, the U-shaped groove portion 83, in which one end surface thereof is opened, is formed along the axial direction of the rod 3. The rod 3 is received in the U-shaped groove portion 83. In the rod receiving portion 80, female threads 88 are formed on each inner wall surface of walls 87, 87 facing each other in the groove portion 83. A set screw 89 is screwed into the female threads 88. The set screw 89 presses down the rod 3 located in the rod receiving portion 80 against the bottom surface of the groove portion 83 of the rod receiving portion 80 so as to connect the rod 3 to the connector 6A. The arm portion 81 is provided on the outer wall surface of one of the pair of wall portions 87, 87 of the rod receiving portion 80, the arm portion 81 being integrally protruded from a position near the bottom surface of the groove portion 83. The arm portion 81 is formed in a circular cross section. The arm portion 81 extends in the direction substantially orthogonal to the axial direction of the rod 3, that is, extends in the direction substantially orthogonal to the direction in which the groove 83 extends.

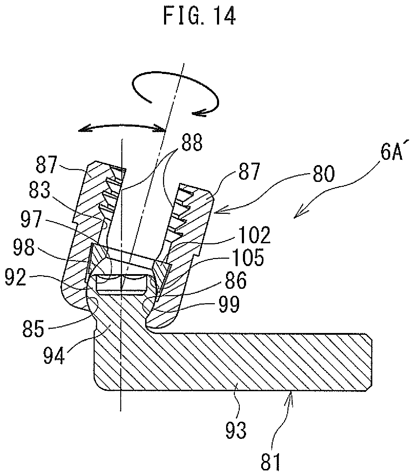

Next, a connector 6A' according to another embodiment will be described with reference to FIG. 13 and FIG. 14. Considering the connector 6A' according to another embodiment, in the rod receiving portion 80, relative to the arm portion 81, the direction where the U-shaped groove portion 83 extends can be optionally oriented. Further, the rod receiving portion 80 is configured to be integrally connectable with the arm portion 81 at optional positions. The connection structure will be described in detail. The arm portion 81 presents an L-shaped formation and includes an engagement head portion 92 to be engaged with the rod receiving portion 80 and an arm main part 93 extending in the direction substantially orthogonal to the rod 3, the arm main part 93 extending from the engagement head portion 92 and connected through a connecting portion 94. The engagement head portion 92 is formed in a spherical shape, and a plane portion 98 is formed on the top thereof. Note that a polygonal concaved portion 97 having a polygonal shape in a plan view is formed on the plane portion 98. The arm main part 93 has a circular cross section.

On the other hand, an insertion hole 85 is formed through the bottom of the groove portion 83 of the rod receiving portion 80. The engagement head portion 92 of the arm portion 81 is engaged in the insertion hole 85. The inner wall surface of the insertion hole 85 has a locking spherical surface 86 for locking a spherical surface 99 provided on the engagement head portion 92 of the arm portion 81. Within the insertion hole 85, a pressing member 102 is engaged slidably in the axial direction. The pressing member 102 has a substantially cylindrical shape, and has a cutout portion 104, which is partially positioned in the circumferential direction, extends in the axial direction and connects the inside with the outside of the pressing member 102. A pressing spherical surface 105 is formed on the inner wall surface of the pressing member 102. The pressing spherical surface 105 of the pressing member 102 comes into contact with the spherical surface 99 of the engagement head portion 92 of the arm portion 81. The rod receiving portion 80 and the arm portion 81 are fitted and assembled in the insertion hole 85 of the rod receiving portion 80 from the engagement head portion 92 side of the arm section 81.

Thus, the rod receiving portion 80 is connected to the arm portion 81 (engagement head portion 92), the rod receiving portion 80 being rotatable around the axial direction of the female threads 88 of the rod receiving portion 80. Further, the rod receiving portion 80 is connected to the arm portion 81 so as to be swingable in all directions within a predetermined angle range. Then, the set screw 89 is screwed into the female threads 88 of the rod receiving portion 80, and the rod 3 is moved toward the arm portion 81 side together with the pressing member 102. With this, the pressing spherical surface 105 of the pressing member 102 presses the spherical surface 99 of the engagement head portion 92 of the arm portion 81 while the spherical surface 99 of the engagement head portion 92 presses down the engagement spherical surface 86 of the insertion hole 85. Accordingly, the rod receiving portion 80 is integrally connected to the arm portion 81 in such a manner that the direction where the groove portion 83 extends is arbitrarily positioned relative to the arm portion 81. Then, at the same time, the rod 3 is integrally connected to the rod receiving portion 80 of the connector 6A'.

Next, the basic spinal correction and fusion method by the spinal fusion system 1A according to the first embodiment will be described.

First, for a plurality of vertebrae selected within a spine-corrective range, the screw 4 is screwed from the back into a vertebral body through the pair of pedicles or unilateral pedicle of a vertebral arch. In the case where the screw 4 cannot be screwed into the pedicle, the first hook portion 41 of the first hook member 5 may be hooked to the pedicle or transverse process of each vertebral arch, from the head side or the caudal side.

Next, a load is applied to each screw 4 that has been screwed into the vertebral body via the pedicle of each vertebral arch, to move each screw 4 in a separate direction along the axial direction of the spine. In this manner, the scoliosis is corrected.

Then, while maintaining this corrective condition, the rod 3, whose appropriate portion is largely bent, is engaged in the rod receiving portion 10 of each screw 4 as well as the rod receiving portion 40 of each first hook member 5. Subsequently, the corresponding set screws 19, 49 are respectively screwed into the female threads 18 of the rod receiving portion 10 of each screw 4 and the female threads 48 of the rod receiving portion 40 of each first hook member 5. In this manner, the rod 3 is temporarily fixed to each screw 4 and each first hook member 5.

Here, in the screw 4, the rod receiving portion 10 is connected to the screw portion 11 in such a manner that the direction where the U-shaped groove portion 13 extends can be optionally oriented (freely swingable). Accordingly, the rod 3 can be easily engaged in the rod receiving portion 10 of each screw 4. Further, also in the first hook member 5, by adopting the first hook member 5' according to another embodiment, since the rod receiving portion 40 is connected to the first hook portion 41 in such a manner that the direction where the U-shaped groove portion 43 extends can be optionally oriented (freely swingable), the rod 3 can be easily engaged in the rod receiving portion 40 of each first hook member 5'.

Next, if necessary, a load may be again applied to each screw 4 in order to separate the screw 4 from another or to move the screw 4 closer to another, along the axial direction of a spine. Thus, scoliosis will be further corrected. Subsequently, the rod 3 is held by special surgical instruments (not shown), and the rod 3 is rotated along the crosswise direction of a patient, thereby correcting the twist of the spine.

Then, the set screws 19 and 49, which have been temporarily fastened to the rod receiving portion 10 of each screw 4 and the rod receiving portion 40 of each first hook member 5, are finally tightened. As a result, in each screw 4, if the rod receiving portion 10 and the screw portion 11 are integrally connected, or if the first hook member 5' of another embodiment is adopted, the rod receiving portion 40 and the first hook portion 41 are integrally connected, in the first hook member 5'. Then, at the same time, the rod 3, each screw 4, and each first hook member 5 (5') are integrally connected.

The rod receiving portion 80 of the connector 6A is engaged at an appropriate position along the axial direction of the rod 3, and the set screw 89 is temporarily tightened to the female threads 88 of the rod receiving portion 80 of the connector 6A. Then, the rod 3 is temporarily tightened in the rod receiving portion 80 of the connector 6A. Subsequently, the arm portion 81 of the connector 6A is rotated about the axis of the rod 3 and held at a position away from the vertebral arch. Then, the second hook portion 51 of the second hook member 7A is hooked to and engaged with lamina from the inside of a spinal canal. While maintaining this condition, the arm portion 81 of the connector 6Ais rotated about the axis of the rod 3 and is engaged in the arm receiving portion 50 of the second hook member 7A. The set screw 59 is then screwed into the female threads 58 of the arm receiving portion 50, and the arm portion 81 of the connector 6A is integrally connected to the arm receiving portion 50 of the second hook member 7A at an optional position along the axial direction of the arm portion 81 of the connector 6A. Moreover, the set screw 89, which has been temporarily tightened to the female threads 88 of the rod receiving portion 80 of the connector 6A, is finally tightened, thereby integrally connecting the connector 6A with the rod 3.

Considering the connector 6A, if adopting the connector 6A' according to another embodiment (see FIG. 13 and FIG. 14), the rod receiving portion 80 is connected to the arm portion 81 in such a manner that the direction where the U-shaped groove portion 83 extends can be optionally oriented (freely swingable). Further, in the second hook member 7A, if adopting the second hook member 7A' according to another embodiment (see FIG. 9 and FIG. 10), the arm receiving portion 50 is connected to the second hook member 51 in such a manner that the direction where the U-shaped groove portion 53 extends can be optionally oriented (freely swingable). Accordingly, the rod 3 and the second hook member 7A' can be easily connected to each other in an integral manner through the connector 6A'. In this regard, when the connector 6A' according to another embodiment is adopted, the set screw 89 is finally tightened to integrally connect the rod 3 to the rod receiving portion 80 of the connector 6A'. At this time, the rod receiving portion 80 and the arm portion 81 of the connector 6A' are integrally connected. Further, when adopting the second hook member 7A' according to another embodiment, the set screw 59 is finally tightened to integrally connect the arm portion 81 of the connector 6A' to the arm receiving portion 50 of the second hook member 7A'. At this time, the arm receiving portion 50 and the second hook portion 51 of the second hook member 7A' are integrally connected.

As described above, in the spinal fusion system 1A according to the first embodiment, in particular, the system 1A has the second hook member 7A (7A') that is connected to the rod 3 through the connector 6A and engaged with the lamina of vertebra. Accordingly, when an external force is applied to the rod 3, the force can be dispersed to the relatively high-strength lamina via the connector 6A and the second hook member 7A (7A'). As a result, the loosening of the rod 3 in a sliding direction with respect to the screw 4 and the first hook member 5 (5') can be suppressed. Moreover, the loosening of the screw 4 in a pull-out direction with respect to a vertebral body can be suppressed. In short, in the spinal fusion system 1A according to the first embodiment, it is possible to strengthen correction and fusion force to a spine so as to eventually advance reliability in stable correction and fusion to the spine.

Moreover, the spinal fusion system 1A according to the first embodiment is applicable to all cases of spinal deformities. However, in recent years, when considering the case of scoliosis of the elderly with progressed osteoporosis, it would be possible that the elderly concurrently have kyphosis. Thus, there is a concern that the screw 4, which has been screwed into a vertebral body via pedicle, may easily come off from a vertebral body. To cope with this problem, the spinal fusion system 1A according to the first embodiment includes the second hook member 7A (7A') that is hooked to and engaged with lamina from the inside of a spinal canal. The second hook member 7A (7A') is simply installed to the relatively high-strength lamina among vertebrae, thereby being able to increase fixing force to a spine. In addition, at present, polyethylene tape is used to enhance fixation force in the spinal fusion system. When using this tape, it must be passed around nerves in the spinal canal, thereby making operative procedures considerably complicated. On the other hand, in the spinal fusion system 1A according to the first embodiment, since the second hook member 7A (7A') is hooked to and engaged with the lamina, not only safety can be further secured, but also operative procedures will be facilitated.

In the spinal fusion system 1A according to the first embodiment, by adopting the first hook member 5' (see FIG. 11) according to another embodiment, since the rod receiving portion 40 is connected to the first hook portion 41 in such a manner that the direction where the U-shaped groove portion 43 extends can be optionally oriented (freely swingable), the rod 3 can be easily engaged in the rod receiving portion 40 (groove portion 43) of each first hook member 41. Further, by adopting the connector 6A' (see FIG. 13 and FIG. 14) according to another embodiment, the rod receiving portion 80 is connected to the arm portion 81 in such a manner that the direction where the U-shaped groove portion 83 extends can be optionally oriented (freely swingable). Yet further, by adopting the second hook member 7A' (see FIG. 9 and FIG. 10) according to another embodiment, the arm receiving portion 50 is connected to the second hook member 51 in such a manner that the direction where the U-shaped groove portion 53 extends can be optionally oriented (freely swingable). Accordingly, the rod 3 and the second hook member 7A' can be easily connected to each other in an integral manner through the connector 6A'.

In short, in spinal deformity correction and fusion surgeries, it is necessary to correct and fix deformed spines by each screw 4, rod 3 and the like. However, operations, which engage the rod 3 with the rod receiving portions 10, 40 of each screw 4 and each of the first hook members 5, will need advanced technical skills. On the other hand, in the spinal fusion system 1A according to the first embodiment, by adopting the first hook member 5', the connector 6A' and the second hook member 7A' according to another embodiment, the rod 3, each screw 4, each first hook members 5', the connector 6A' and the second hook member 7A' can be easily connected to each other. As a result, operative procedures in spinal deformity correction and fusion surgeries no longer need any of the advanced techniques, so that the operation time can be shortened so as to reduce the burden on patients.

In the spinal fusion system 1A according to the first embodiment, by making the outer diameter of the rod 3 and the outer diameter of the arm portion 81 of the connector 6A (6A') as the same, it is possible that the hook member 5' according to another embodiment (see FIG. 11) and the second hook member 7A' according to another embodiment (see FIG. 9) are made as common parts, eventually both being the same member. This can eliminate the need for operators to appropriately select the corresponding hook members 5' and 7A' during operations, thereby contributing to shortening the operation time. In the spinal fusion system 1A according to the first embodiment, the second hook member 7A (7A') is hooked to and engaged with lamina from the inside of the spinal canal of a vertebral arch. In this regard, it may be hooked to and engaged with the pedicle or transverse process, etc. of a vertebral arch.

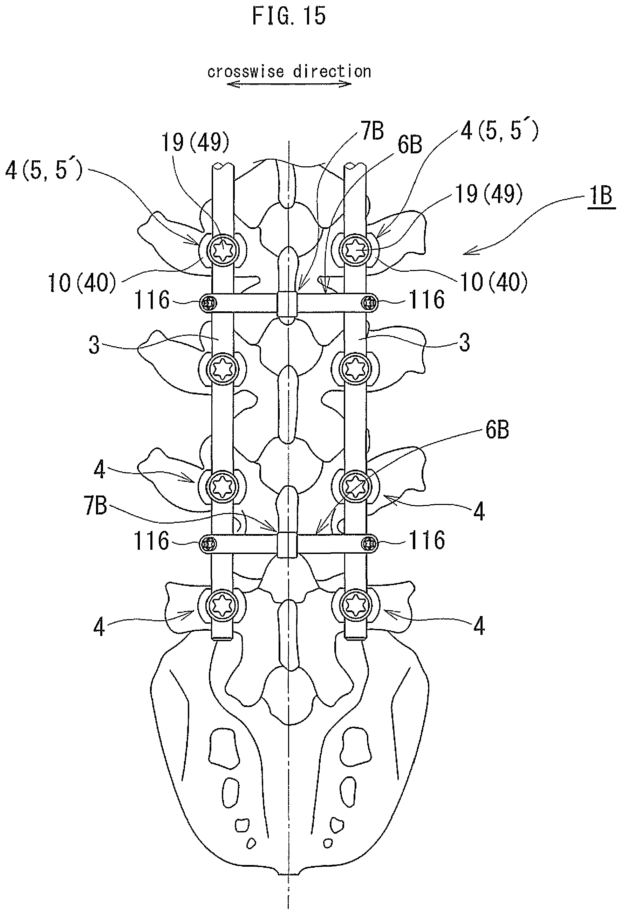

Next, the spinal fusion system 1B according to the second embodiment will be described in detail with reference to FIG 15 to FIG. 17. In the explanation of the spinal fusion system 1B according to the second embodiment, only differences from the spinal fusion system 1A according to the first embodiment will be described. The spinal fusion system 1B according to the second embodiment differs from the spinal fixation system 1A according to the first embodiment in the connector 6A (6A') and the second hook member 7A (7A') adopted in the spinal fusion system 1A according to the first embodiment.

The connector 6B adopted in the spinal fusion system 1B according to the second embodiment includes an arm portion 110 extending in the direction substantially orthogonal to the rod 3, and a rod receiving portion 111 provided at both longitudinal ends of the arm portion 110 and having a groove portion 113 for opening a second hook portion 121 side of the second hook member 7B. The arm portion 110 bridges the pair of rods 3, 3. The arm portion 110 has a substantially rectangular cross section. The groove 113 of each rod receiving portion 111 is formed along the axial direction of the rod 3. The pair of rods 3, 3 are introduced into these grooves 113, 113, respectively. In each of the rod receiving portions 111, female threads 115 penetrating toward the groove portion 113 is formed. A fixing screw 116 is screwed into the female threads 115. The tip of the fixing screw 116 is formed in a conical shape.

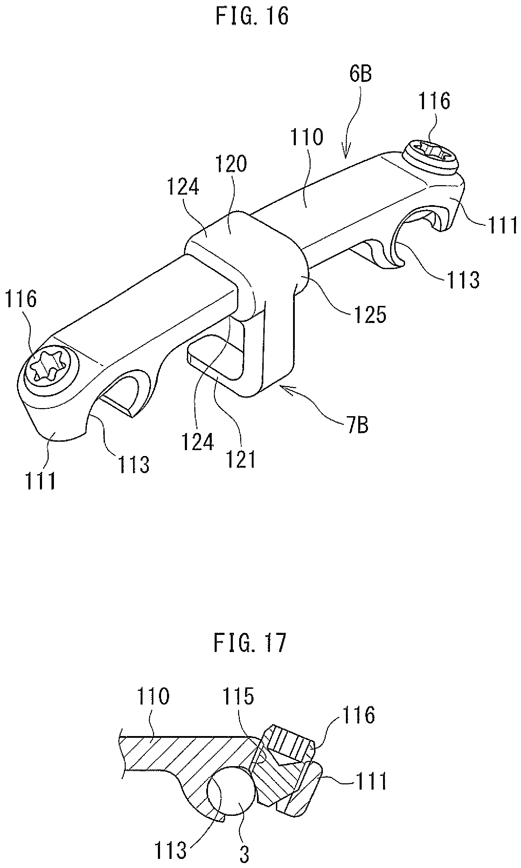

Then, as shown in FIG. 17, with the rod 3 engaged in the groove 113 of the rod receiving portion 111, the fixing screw 116 is screwed into the female threads 115 of the rod receiving portion 111. With this, the taper surface of the tip of the fixing screw 116 presses the rod 3 against the inner wall surface of the groove 113. The rod 3 can be thus integrally connected to the groove 113 of the rod receiving portion 111. The groove portion 113 provided in the rod receiving portion 111 of the connector 6B is formed so as to open the second hook portion 121 side of the second hook member 7B. Accordingly, when the arm portion 110 of the connector 6B is connected to the pair of rods 3, 3, those rods 3,3 can be easily engaged in the groove portions 113, 113 of the rod receiving portions 111, 111.

The second hook member 7B includes a sliding main body 120 slidably connected in the axial direction of the arm portions 110 and the second hook portion 121 extending integrally from the sliding main body 120 and hooked to and engaged from lamina of a vertebral arch from a spinal canal. The sliding main body 120 includes a pair of horizontal plate parts 124, 124 facing each other and a vertical plate part 125 integrally connected to the ends of the pair of horizontal plate parts 124, 124. The sliding main body 120 is thus formed in an approximately U-shape. The arm portion 110 is slidably connected in the axial direction between the opposed horizontal plate parts 124, 124 of the sliding main body 120. The distance between the opposed horizontal plate parts 124, 124 of the sliding main body 120 is set to be larger than the thickness of the arm portion 110 such that the arm portion 110 can slide between the horizontal plate parts 124, 124. At the tip of one horizontal plate part 124 among the opposed horizontal plate parts 124, 124 of the sliding main body 120, a stopper (not shown in figures) projecting toward the tip of the other horizontal plate part 124 is provided. The distance between this stopper and the tip of the other horizontal plate part 124 is set slightly smaller than the thickness of the arm portion 110.

When connecting the sliding main body 120 to the arm portion 110, the opposed horizontal plate parts 124, 124 of the sliding main body 120 are elastically deformed so as to separate one from another. Then, the arm portion 110 is inserted between the stopper of one horizontal plate part 124 and the other horizontal plate part 124 so as to connect therebetween. As a result, the sliding main body 120 will not fall from the arm portion 110 by means of the stopper, so that the sliding main body 120 is supported by the arm portion 110, the sliding main body 120 being slidable along the axial direction of the arm portion 110. The second hook portion 121 is integrally connected to the outer wall surface of the vertical plate part 125 of the sliding main body 120. The second hook portion 121 is formed in a substantially L-shape so as to be hooked to and engaged with lamina from the inside of a spinal canal. Although not shown in figures, in the sliding main body 120, the following structure may be possibly provided: a female thread is provided through the horizontal plate part 125 opposite to the second hook portion 121 side, and a set screw is screwed into the female thread. With this, the sliding main body 120 can be fixed at an optional position on the arm portion 110, by means of pressure from the set screw.

In the spinal fusion system 1B according to the second embodiment as described above, the connector 6B includes the arm portion 110 extending in the direction substantially orthogonal to the rod 3, and the rod receiving portion 111 provided on both longitudinal ends of the arm portion 110 and having the groove portion 113 for opening the second hook portion 121 side of the second hook member 7B. With this, the sliding main body 120 of the second hook member 7B is then slidably connected along the axial direction of the arm portion 110. Further, the pair of rods 3, 3 are integrally connected to the groove portions 113, 113 of each of the rod receiving portions 111, 111 of the arm portion 110. Thereby, in addition to operational effects by the spinal fusion system 1A according to the first embodiment, rigidity in twisting directions with respect to the pair of rods 3, 3 can be strengthened, and correction and fusion force to a spine is further enhanced. Thus, reliability in stable correction and fusion to a spine can be further improved.

Note that, as described above, the spinal fusion systems 1A, 1B according to the first and second embodiments have been adopted as a spinal deformity correction and fusion system that fixes the relatively long range of a spine, in order to correct and fix spinal deformity such as scoliosis or kyphosis. However, those systems may be adopted as a system that fixes and stabilizes spine in the relatively short range of a spine when considering other spine diseases such as spondylolisthesis or vertebral fracture.

### [Explanation of symbols]

1A, 1B: spinal fusion system, 3: rod, 4: screw (supporting element), 5, 5': first hook member (supporting element), 6A, 6A', 6B: connector, 7A, 7A', 7B: second hook member, 40: rod receiving portion, 41: first hook portion, 43: groove portion, 50: arm receiving portion, 51: second hook portion, 53: groove portion, 80: rod receiving portion, 81: arm portion, 83 groove portion

## Claims

1. A spinal fusion system including:
each supporting element that supports each vertebra constituting a spine; and
a rod that extends along an axial direction of the spine and is connectable to the supporting element, the spinal fusion system further including:
a connector that is detachably connectable to the rod; and
a hook member that is connectable to the connector and engageable with a vertebral arch.

2. The spinal fusion system according to claim 1, wherein the connector has an arm portion that extends in a direction approximately orthogonal to an axial direction of the rod, and the hook member is detachably installed at an optional position along an axial direction of the arm portion.

3. The spinal fusion system according to claim 2, wherein the hook member includes an arm receiving portion that has a groove portion in which to receive the arm portion of the connector, and a hook portion that is connectable to the arm receiving portion and is engageable with the vertebral arch, wherein the arm receiving portion is configured to be integrally connectable with the hook portion at an optional position in such a manner that a direction where the groove portion extends is allowed to be optionally oriented.

4. The spinal fusion system according to claims 2 or 3, wherein the connector is provided with a rod receiving portion that is connectable to the arm portion and has a groove portion in which to receive the rod, and
the rod receiving portion is configured to be integrally connectable with the arm portion at an optional position in such a manner that a direction where the groove portion extends is allowed to be optionally oriented.

5. The spinal fusion system according to claim 1, wherein the connector has an arm portion that extends in a direction approximately orthogonal to an axial direction of the rod, and the hook member is slidably connectable along an axial direction of the arm portion.

6. The spinal fusion system according to any one of claims 2 to 5, wherein the pair of rods are arranged on both left and right sides of a spinous process of each vertebral arch, and both longitudinal ends of the arm portion are each detachably connectable to the pair of rods.

7. A spinal fusion system including:
a rod that extends along an axial direction of a spine; and
a first hook member that is detachably installed to the rod and is engageable with a vertebral arch, wherein the first hook member includes a rod receiving portion that has a groove portion in which to receive the rod, and a hook portion that is connectable to the rod receiving portion and is engageable with the vertebral arch, and
the rod receiving portion is configured to be integrally connectable to the hook portion at an optional position in such a manner that a direction where the groove portion extends is allowed to be optionally oriented.

8. The spinal fusion system according to claim 7, the spinal fusion system including:
a connector that is detachably connectable to the rod, and
a second hook member that is connectable to the connector and engageable with a vertebral arch.
